# EUROPEAN PATENT APPLICATION

(11) **EP 1 598 036 A1**
(43) Date of publication of application: **23.11.2005**
(21) Application number: 04076474.8
(22) Date of filing: 19.05.2004
(51) Int. Cl.: A61F 13/44

(54) **System comprising a surgical gauze and detecting means**

(71) Applicant: AutoMedic B.V., 5644 JB Eindhoven (NL)
(72) Inventor: Marinelli, Werner Alexander, 5644 JB Eindhoven (NL)
(74) Representative: Verhees, Godefridus Josephus Maria

(57) **Abstract**

A surgical gauze (1) is provided with a RF-ID tag (2). The RF-ID tag consists of a small integrated circuit (IC) and an antenna connected to the IC. The antenna is tuned to an appropriate radio frequency. When the RF-ID tag is put near to a RF-ID reader, it will receive the radio-frequency radiation and return at the same radio frequency its own information back to the reader or master transponder.

In case of large gauzes, where such gauze may be cut into two or more pieces during surgical usage, two or more RF-ID tags may be applied to that gauze to ensure that each piece of gauze cut from the original, one-piece, large gauze can still be found and identified.

Each RF-ID tag bears both a unique number, by which it is identified, and some specific information, which can be stored in the writable memory of the RF-ID tag. For example, the specific information may contain the size of the gauze and/or the fact whether there is more than one tag in that gauze.

## Description

### BACKGROUND OF THE INVENTION:

### Field of the invention

The invention relates to a comprising a surgical gauze provided with a detectable means that can be detected without making contact to said means, as well as detecting means for detecting the surgical gauze, which detecting means use radiation to detect the detectable means. These gauzes may not remain in a patient after the operation because it can cause malfunctioning of the patient or even lead to death.

### Prior art

A similar system is generally known. The gauze of the known system comprises a yarn containing metal threads and the detecting means consists of an x-ray or roentgen device. The known gauzes used in a surgical environment are usually counted before being applied and after being used. Currently, this counting is not supported by any automated system and relies on the accurateness of the surgical assistants. Especially at unexpectedly long operations where another crew replaces the crew of surgical assistants, the conventional counting method can lead to miscounting.

In case a gauze is missing, it can be looked for using a detecting means such as an x-ray (or roentgen) device, mostly a mobile version that must be brought into the surgical environment from another place. In this case, part of the gauze must consist of yarn containing metal threads, which can be detected by the x-ray device.

However, these metal-containing threads sometimes are pulled out of the gauze when such gauze is cut into pieces during usage and the threads are sticking to the first piece of that gauze being taken out of the patient. This x-ray method has the disadvantages of needing a (mobile) x-ray device for searching a missing gauze in the patient and the fact that x-rays are detrimental to the status of the human body.

### Summary of the invention

An objective of the invention is to provide a system of the type described in the preamble with which gauzes can be counted and detected without having the disadvantages of the known system. For this purpose, the system according to the invention is characterised in that the detectable means are such that they can be detected with the use of radiation that is non-detrimental to the human body. An embodiment of the system according to the present invention is characterized in that the detectable means comprises at least one radio-frequent identification device (RF-ID). The device can be discovered and identified with radio frequency, which is not detrimental to the human body like x-ray or roentgen.

Using non-collision RF-IDs and appropriate RF-frequencies, all tags can be read simultaneously without need for the user to put the gauzes (plus RF-IDs) one by one near the detecting device such as an antenna of a master transponder. The RF-ID can be small and flexible so that it will not essentially change the characteristics of the gauze. Further the RF-ID can be sterile or sterilized.

A further embodiment of the system according to the invention is characterised in that the radio-frequent identification device comprises an integrated circuit, bearing a unique number and preferably having memory containing information about the gauze on which it is attached, and an antenna which is connected to the integrated circuit. Each gauze can be identified with unique number and specific information in memory.

Preferably the detectable means comprises at least one further radio-frequent identification device. In case of large gauzes, where such gauze may be cut into two or more pieces during surgical usage, two or more RF-IDs may be applied to that gauze to ensure that each piece of gauze cut from the original, one-piece, large gauze can still be found and identified. Each RF-ID preferably bears both a unique number, by which it is identified, and some specific information, which can be stored in the writable memory of the RF-ID. For example, the specific information may contain the size of the gauze and/or the fact whether there is more than one tag in that gauze.

Yet a further embodiment of the system according to the invention is characterised in that the detecting means comprises a radio-frequency transmitter and receiver. Preferably the detecting means further comprises a computer, which is connected to the radio-frequent receiver. The computer comprises a program that at the beginning will identify all gauzes and RF-IDs and store this information in a suitable database and easily readable layout. When the gauzes will be put back in or near the radio-frequency transmitter and receiver, after they have been used, the program will compare the RF-IDs discovered and identified with those of the original list and will detect whether any gauze or piece of gauze is missing. If so, it will clearly communicate this to the user of the system, possibly with a kind of alarm and the specific information of the missing gauze(s), when this was written into the related memory. When one or more gauzes and RF-IDs are missing, they must be found.

The invention also relates to a surgical gauze provided with detectable means for use in a system according to the invention. With regard to the gauze, the invention is characterised in that the detectable means are such that they can be detected with the use of radiation that is non-detrimental to the human body. Preferably the detectable means comprises a radio-frequent identification device. An embodiment of the surgical gauze according to the present invention is characterized in that the radio-frequent identification device comprises an integrated circuit, bearing a unique number and preferably having memory containing information about the gauze on which it is attached, and an antenna, which is connected to the integrated circuit. Further, the detectable means preferably comprises at least one further radio-frequent identification device.

The invention further relates to a detecting means for use in a system according to the invention. With regard to the detecting means, the invention is characterised in that the detecting means comprises a radio-frequent transmitter and receiver. Preferably, the detecting means further comprises a computer, which is connected to the radio-frequent receiver.

The invention also relates to a method for using surgical gauzes provided with detectable means, which gauzes will be counted before and after the surgery. With regard to the method, the invention is characterised in that before the surgery, gauzes, which are provided with a radiation identification device, will be put close to a radiation transmitter and receiver, whereby the radiation used is non-detrimental to the human body, and a computer connected to the receiver will count the gauzes, and after the surgery the gauzes will again be put close to a radiation transmitter and receiver and the computer shows whether gauzes are missing or not.

Preferably the radiation identification device is a radio-frequent identification device and the radiation transmitter and receiver preferably uses radio-frequent radiation. Further the computer not only counts the gauzes but preferably also identifies the gauzes by reading information from the memory of the integrated circuit and shows particulars about the missing gauzes. A RF-ID list is made up before using the gauzes. This list is the counting reference for after the gauzes have been used and put back to the RF transmitter and receiver. The system will exactly determine whether one or more gauzes is missing and if so, which one(s).

### Brief description of the drawings

The invention will be elucidated more fully below on the basis of drawings in which embodiments of the system according to the invention are shown. In these drawings:
Figure 1 shows an embodiment of a surgical gauze in accordance with the invention;
Figure 2 shows a container for storage and disposal of the gauzes used;
Figure 3 shows a schematically drawing of a patient with a master transponder for finding one or more used gauzes; and
Figure 4 shows the use of a handheld RF-ID tag reader as an alternatively to the master transponder of FIG. 3.

### Detailed description of the drawings

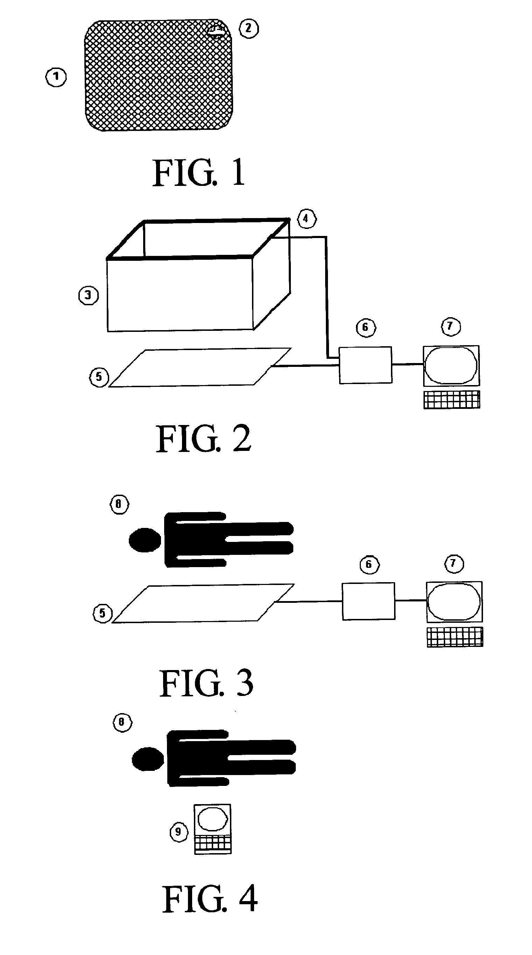

FIG. 1 is a drawing of surgical gauze (I), usually sterilized before being packed and used. To the gauze, a RF-ID tag (2) is applied. The RF-ID tag consists of a small integrated circuit (IC) bearing a unique number and mostly some memory where information can be written into or read from. This IC is connected to an antenna which is tuned to the appropriate radio frequency, for example in the range of 13,56 MHz. When the RF-ID tag is put near to a RF-ID reader or master transponder, it will receive the radio-frequency radiation and return at the same radio frequency its own information back to the reader or master transponder.

The chosen radio-frequency must assure an appropriate working range (typically 10-50 cm) and should not be absorbed too much by fluid containing embodiments like gauzes with blood or the interior of a patient under operation.

The RF-ID tags, its IC and antenna, must be packed in such way that they are sterile or can be made sterile and do not interact with (human body) fluids. The RF-ID tag can for example be put into a small, flat plastic packing (e.g. with a lamination process), which fully protects the IC and antenna, is flexible and resistant to bending, fluids, temperatures and pressures under usage and under sterilization conditions.

As the RF-ID tags must be applied to a gauze, the packing of the RF-ID tag should be such that it can be fixed to the gauze without the risk of getting lost during normal usage. Therefore, the tag can be sewed to the gauze or put into a "chamber" in the gauze where it consists of two layers and the tag is put in between.

In case of large gauzes, where such gauze may be cut into two or more pieces during surgical usage, two or more RF-ID tags may be applied to that gauze to ensure that each piece of gauze cut from the original, one-piece, large gauze can still be found and identified.

Each RF-ID tag bears both a unique number, by which it is identified, and some specific information, which can be stored in the writable memory of the RF-ID tag. For example, the specific information may contain the size of the gauze and/or the fact whether there is more than one tag in that gauze.

FIG. 2 shows a container (3) for storage and disposal of the gauzes used. This can be of any size and form suited for the surgical needs. Before using the gauzes, they can be put in or near the container, even without taking them out of the original packing, preserving the sterilized status of the gauzes. After using the gauzes, they can be put into the container.

Any gauze being close to the container will be discovered and identified by the RF-ID master transponder, reading the information of the RF-ID tags applied to the gauzes.

The RF-ID master transponder consists of an antenna, a controller (6) and a software program running on a computer (7). The antenna can be shaped like a ring at the top edge of the container (4) or like a flat plate underneath the place where the gauzes are put (5). The antenna can have an optimized form to distinguish all RF-ID tags in all kind of locations, directions and positions related to each other, so to optimize the discovery and identification of all the gauzes and RF-ID tags.

The computer program (7) will at the beginning identify all gauzes and RF-ID tags and stores this information in a suitable database and easily readable layout. When the gauzes will be put back in or near the RF-ID master transponder, after they have been used, the program will compare the RF-ID tags discovered and identified with those of the original list and will detect whether any gauze or piece of gauze is missing. If so, it will clearly communicate this to the user of the system, possibly with a kind of alarm and the specific information of the missing gauze(s), when this was written into the related memory. When one or more gauzes and RF-ID tags are missing, they must be found, see figs. 3 and 4.

FIG. 3 is an schematically drawing of a patient (8) for whom one or more gauzes were used, for example during an operation. When one or more gauzes and RF-ID tags must be found in or near the patient, a mobile or handheld RF-ID tag reader or master transponder can be used. A possible implementation of a mobile RF-ID tag master transponder can be an antenna in the form of a flat plate (5), which can be put near the patient and which is connected to the controller (6) and computer (7) for discovery and identification of the missing gauze(s).

Alternatively to the master transponder depicted and described in FIG. 3, a handheld RF-ID tag reader as shown in FIG. 4 can be used. It must be verified whether such handheld reader can reach all possible locations to discover the missing gauze(s) in or near the patient.

Although in the above the invention is explained on the basis of the drawings, it should be noted that the invention is in no way limited to the embodiments shown in the drawings. The invention also extends to all embodiments deviating from the embodiments shown in the drawings within the context defined by the claims.

## Claims

1. System comprising a surgical gauze provided with a detectable means that can be detected without making contact to said means, as well as detecting means for detecting the surgical gauze, which detecting means use radiation to detect the detectable means, **characterized in that** the detectable means are such that they can be detected with the use of radiation that is non-detrimental to the human body.

2. System according to claim 1, **characterized in that** the detectable means comprises at least one radio-frequent identification device.

3. System according to claim 2, **characterized in that** the radio-frequent identification device comprises an integrated circuit, bearing a unique number and preferably having memory containing information about the gauze on which it is attached, and an antenna which is connected to the integrated circuit.

4. System according to claim 2 or 3, **characterized in that** the detectable means comprises at least one further radio-frequent identification device.

5. System according to claim 2, 3 or 4, **characterized in that** the detecting means comprises a radio-frequent transmitter and receiver.

6. System according to claim 5, **characterized in that** the detecting means further comprises a computer which is connected to the radio-frequent receiver.

7. Surgical gauze provided with detectable means for use in a system according to one of the preceding claims, **characterized in that** the detectable means are such that they can be detected with the use of radiation that is non-detrimental to the human body.

8. Surgical gauze according to claim 7, **characterized in that** the detectable means comprises a radio-frequent identification device.

9. Surgical gauze according to claim 8, **characterized in that** the radio-frequent identification device comprises an integrated circuit, bearing a unique number and preferably having memory containing information about the gauze on which it is attached, and an antenna which is connected to the integrated circuit.

10. Surgical gauze according to claim 8 or 9, **characterized in that** the detectable means comprises at least one further radio-frequent identification device.

11. Detecting means for use in a system according to one of the preceding claim 1 to 6, **characterized in that** the detecting means comprises a radio-frequent transmitter and receiver.

12. Detecting means according to claim 11, **characterized in that** the detecting means further comprises a computer which is connected to the radio-frequent receiver.

13. Method for using surgical gauzes provided with detectable means, which gauzes will be counted before and after the surgery, **characterized in that** before the surgery, gauzes, which are provided with a radiation identification device, will be put close to a radiation transmitter and receiver, whereby the radiation used is non-detrimental to the human body, and a computer connected to the receiver will count the gauzes, and after the surgery the gauzes will again be put close to a radiation transmitter and receiver and the computer shows whether gauzes are missing or not.

14. Method according to claim 13, **characterized in that** the radiation identification device is a radio-frequent identification device and that the radiation transmitter and receiver uses radio-frequent radiation.

15. Method according to claim 13 or 14, **characterized in that** the computer not only counts the gauzes but also identifies the gauzes by reading information from the memory of the integrated circuit and shows particulars about the missing gauzes.
